(19) Europäisches Patentamt
European Patent Office
Office européen des brevets

(11) EP 0 624 086 B1

(12) **FASCICULE DE BREVET EUROPEEN**

(45) Date de publication et mention de la délivrance du brevet:
**20.03.1996 Bulletin 1996/12**

(21) Numéro de dépôt: **93902340.4**

(22) Date de dépôt: **17.12.1992**

(51) Int. Cl.$^{6}$: **A61K 7/00**

(86) Numéro de dépôt international: **PCT/FR92/01195**

(87) Numéro de publication internationale:
**WO 93/14738 (05.08.1993 Gazette 1993/19)**

(54) **COMPOSITION COSMETIQUE DESTINEE A ETRE ADDITIONNEE A L'EAU D'UN BAIN**
KOSMETISCHES MITTEL ALS BADERWASSERZUSATZ
COSMETIC COMPOSITION FOR ADDING TO BATH WATER

(84) Etats contractants désignés:
**DE ES FR GB IT**

(30) Priorité: **29.01.1992 FR 9200939**

(43) Date de publication de la demande:
**17.11.1994 Bulletin 1994/46**

(73) Titulaire: **L'OREAL**
**F-75008 Paris (FR)**

(72) Inventeurs:
- **HANSENNE-RICHOUX, Isabelle**
  **F-75017 Paris (FR)**
- **MAURIN, Véronique**
  **F-75005 Paris (FR)**

(74) Mandataire: **Peuscet, Jacques et al**
**SCP Cabinet Peuscet et Autres,**
**68, rue d'Hauteville**
**F-75010 Paris (FR)**

(56) Documents cités:
**EP-A- 0 130 577** **EP-A- 0 158 441**
**DE-A- 4 021 083**

- **Chemical Abstracts, vol. 104, no. 7, 1986, (Columbus, Ohio, US), voir page 375, abrégé no. 74794z, & JP,A,60153938 (POLA CHEM. IND.) 13 août 1985, voir abrégé**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.9.13/3.4

## Description

La présente invention concerne une composition cosmétique destinée à être additionnée à l'eau d'un bain pour le soin de la peau.

On sait qu'il est possible de préparer des vésicules à partir de certains lipides amphiphiles, c'est-à-dire de lipides dont les molécules sont constituées d'une partie lipophile et d'une partie hydrophile. Les vésicules obtenues sont délimitées par une membrane de phase lipidique formée d'un feuillet ou de plusieurs feuillets concentriques, ladite membrane définissant un volume interne fermé où est encapsulée une phase, dite phase encapsulée. Les vésicules sont généralement préparées sous forme de dispersion dans une phase aqueuse, dite phase de dispersion.

Les lipides amphiphiles de la phase lipidique peuvent être des lipides ioniques tels que les lécithines naturelles (lécithine d'oeuf, de soja) ou synthétiques (dipalmitoyllécithine, lécithine d'oeuf hydrogénée). L'utilisation d'une telle phase est décrite dans DE-A-4 021 083. Les lipides amphiphiles peuvent également être des lipides non-ioniques tels que des dérivés de polyglycérol linéaires ou ramifiés, des éthers de polyglycérol linéaires ou ramifiés, des alcools gras polyoxyéthylénés, des stérols polyoxyéthylénés, des éthers de polyols, des esters de polyols oxyéthylénés ou non, des glycolipides, ou certains hydroxyamides.

La phase lipidique peut, de façon connue, contenir un ou plusieurs lipides amphiphiles ioniques, un ou plusieurs lipides amphiphiles non-ioniques ou, à la fois, au moins un lipide amphiphile ionique et au moins un lipide amphiphile non-ionique.

Il est bien connu d'introduire dans la phase lipidique des additifs, qui ont pour fonction de diminuer la perméabilité des membranes vésiculaires et d'améliorer la stabilité desdites membranes. Ces additifs, sont généralement choisis parmi les stérols et les stabilisants anioniques.

De nombreux procédés de fabrication de dispersions vésiculaires de lipides amphiphiles ioniques et/ou non-ioniques sont connus. Divers modes de préparation sont, par exemple, décrits dans "Les liposomes en biologie cellulaire et pharmacologie", Editions INSERM/John Libbey Emotext, 1987 pages 6 à 18 et dans le brevet français 2 315 991.

Il a également été proposé dans EP-A 130 577 d'utiliser, dans un procédé de fabrication de vésicules de lipides ioniques, certains alkylèneglycols et alkyléthers de ces alkylèneglycols comme solvant. Mais, dans les dispersions de vésicules obtenues, le solvant se trouve essentiellement à l'intérieur des vésicules ; seules, des traces se trouvant dans la phase aqueuse de dispersion.

On sait qu'une composition cosmétique destinée à être introduite dans l'eau d'un bain, doit présenter des propriétés d'autodispersion telles qu'elles permettent son mélange facile avec l'eau du bain et, par conséquent, l'obtention rapide d'une répartition uniforme de ladite composition dans toute l'eau du bain. De plus, la composition cosmétique doit être stable dans le temps, c'est-à-dire au cours du stockage.

Selon la présente invention, on a trouvé que l'addition en quantités déterminées relativement élevées de certains alkylèneglycols ou d'alkyléthers de ces alkylèneglycols dans la phase aqueuse d'une dispersion de vésicules de lipide(s) amphiphile(s) ionique(s) et/ou non-ionique(s) permettait d'obtenir des compositions cosmétiques pour le bain qui, à la fois, ont de bonnes propriétés d'autodispersion et sont stables au cours du stockage.

La présente invention a, par conséquent, pour objet une composition cosmétique destinée à être additionnée à l'eau d'un bain pour le soin de la peau, comprenant, dans une phase aqueuse de dispersion, une dispersion de vésicules, dont la membrane est constituée par une phase lipidique contenant au moins un lipide amphiphile ionique et/ou au moins un lipide amphiphile non-ionique, caractérisée par le fait que la phase aqueuse de dispersion contient au moins un composé de formule

$$R'\text{-}O\text{-}(RO)_n\text{-}R'' \qquad (I)$$

formule dans laquelle R est un radical alkylène, linéaire ou ramifié, en $C_2$-$C_3$, R' et R", identiques ou différents, sont H ou un radical alkyle en $C_1$-$C_4$ et n est un nombre entier compris entre 1 et 3 inclus, le rapport pondéral entre la phase lipidique des vésicules et l'ensemble du (ou des) composé(s) de formule (I) contenus dans la phase de dispersion étant compris entre 0,25 et 2,5. Ce rapport pondéral est, de préférence, compris entre 0,5 et 1,5.

La demanderesse a, en effet, constaté que, lorsque le rapport pondéral [phase lipidique/composé(s) de formule (I)] est inférieur à 0,25, la composition n'est plus stable au stockage et que, lorsque ce rapport est supérieur à 2,5, les compositions n'ont plus des propriétés convenables d'autodispersion dans le bain.

Les composés de formule (I) sont donc des mono-, di- ou trialkylèneglycols ou des mono- ou diéthers de mono-, di- ou trialkylèneglycol.

Les composés de formule (I) sont, de préférence, choisis dans le groupe formé par l'éthylèneglycol, le diéthylèneglycol, le propylèneglycol et le dipropylèneglycol, les éthers monométhylique, monoéthylique ou monobutylique de l'éthylèneglycol et du diéthylèneglycol et les monométhyléthers du propylèneglycol et du dipropylèneglycol.

Au moins un composé de formule (I) peut également être contenu dans la phase aqueuse encapsulée par les membranes vésiculaires.

Avantageusement, la composition selon l'invention contient de 0,5 à 20 % et, de préférence, de 2 à 18 % en poids de composé(s) de formule (I) par rapport au poids total de la composition.

Selon l'invention, tous les lipides amphiphiles ioniques et/ou non-ioniques susceptibles de former des vésicules stables, seuls ou en mélange avec des additifs ayant pour fonction de diminuer la perméabilité des membranes des vésicules et d'améliorer leur stabilité, peuvent être utilisés pour constituer les membranes lipidiques des vésicules des compositions selon l'invention. On utilise, de préférence, les lipides amphiphiles non-ioniques.

Parmi les lipides amphiphiles non-ioniques utilisables selon l'invention, on peut citer :

(1) les dérivés de polyglycérol, linéaires ou ramifiés, de formule

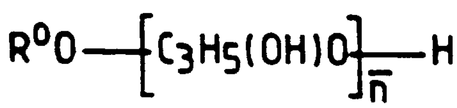 (II)

formule (II) dans laquelle :

- $-C_3H_5(OH)O-$ est représenté par les structures suivantes prises en mélange ou séparément :
  $-CH_2CHOHCH_2O-$ ,

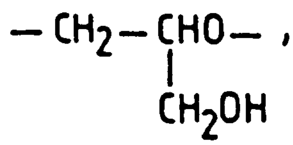 , 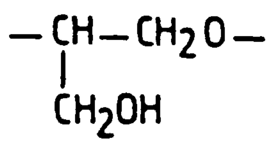

- $\bar{n}$ est une valeur statistique moyenne comprise entre 1 et 6 ;
- $R^o$ représente :

  (a) une chaîne aliphatique, linéaire ou ramifiée saturée ou insaturée, contenant de 12 à 30 atomes de carbone ; ou des radicaux hydrocarbonés des alcools de lanoline ; ou les restes d'alpha-diols à longue chaîne ;
  (b) un reste $R^1CO$, où $R^1$ est un radical aliphatique, linéaire ou ramifié, en $C_{11}$-$C_{29}$ ;
  (c) un reste
  $R^2\{OC_2H_3(R^3)\}$
  où :

  - $R^2$ peut prendre la signification (a) ou (b) donnée pour $R^O$ ;
  - $-OC_2H_3(R^3)-$

  est représenté par les structures suivantes, prises en mélange ou séparément :

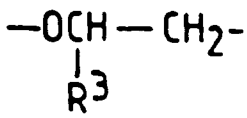 et 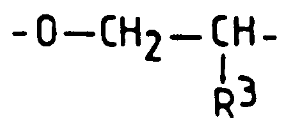

où $R^3$ prend la signification (a) donnée pour $R^o$ ;
(2) les éthers de polyglycérol, linéaires ou ramifiés, comportant deux chaînes grasses ;
(3) les alcools gras polyoxyéthylénés et les stérols et phytostérols polyoxyéthylénés ;

(4) les éthers de polyols ;

(5) les esters de polyols, oxyéthylénés ou non ;

(6) les glycolipides d'origine naturelle ou synthétique et notamment les éthers et les esters de glucose ;

(7) les hydroxyamides représentés par la formule :

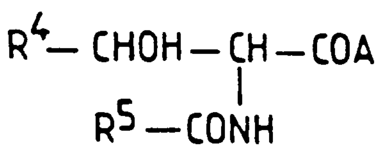

(III)

formule (III) dans laquelle :

- $R^4$ désigne un radical alkyle ou alcényle en $C_7$-$C_{21}$ ;
- $R^5$ désigne un radical hydrocarboné, saturé ou insaturé, en $C_7$-$C_{31}$ ;
- COA désigne un groupement choisi parmi les deux groupements suivants :

  - un reste

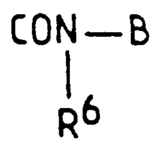

où :

. B est un radical alcoyle dérivé d'amines primaires ou secondaires, mono- ou polyhydroxylées ; et

. $R^6$ désigne un atome d'hydrogène ou un radical méthyle, éthyle ou hydroxyéthyle ; et

  - un reste -COOZ, où Z représente le reste d'un polyol en $C_3$-$C_7$.

(8) les dérivés du glycérol décrits dans la demande de brevet PCT n°91/00889 déposée le 13 Novembre 1991 et répondant à la formule :

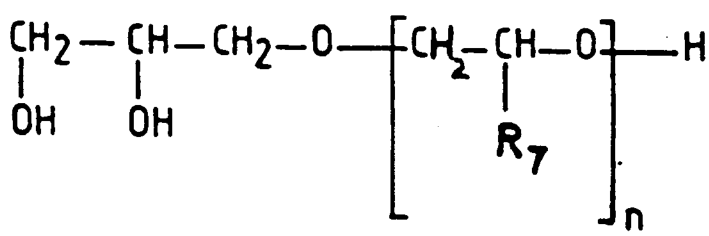

(IV)

formule (IV) dans laquelle $R_7$ représente un radical alkyle linéaire en $C_{14}$-$C_{18}$ ou un groupement $-CH_2A$ dans lequel A est $-OR_{14}$, $R_{14}$ représentant un radical alkyle linéaire en $C_{10}$ à $C_{18}$ et, de préférence en $C_{16}$, et n représente ou bien une valeur statistique moyenne supérieure à 1 et au plus égale à 3, ou bien, lorsque $R_7$ représente $-CH_2A$, une valeur égale à 2.

Parmi les lipides amphiphiles ioniques utilisables, on peut citer :

- les phospholipides naturels tels que la lécithine d'oeuf ou de soja, ou la sphyngomyéline, les phospholipides modifiés par voie chimique ou enzymatique, tels que la lécithine hydrogénée et les phospholipides de synthèse tels que la dipalmitoylphosphatidylcholine ;

- des composés anioniques tels que ceux représentés par la formule

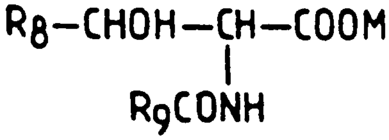

(V)

formule (V) dans laquelle :

. $R_8$ représente un radical alkyle ou alcényle en $C_7$-$C_{21}$

• $R_9$ représente un radical hydrocarboné, saturé ou insaturé en $C_7$-$C_{31}$, et
• M représente H, Na, K, $NH_4$ ou un ion ammonium substitué dérivé d'une amine,

- les gangliosides,
- les composés cationiques dérivés d'ammonium quaternaire répondant à la formule :

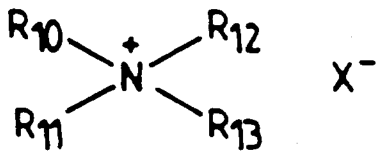

(VI)

avec $R_{10}$ et $R_{11}$, identiques ou différents, représentant des radicaux alkyle en $C_{12}$ - $C_{20}$ et $R_{12}$ et $R_{13}$, identiques ou différents, des radicaux alkyle en $C_1$ - $C_4$ ; et
- des lipides polymérisables, comme ceux décrits par Ringsdorf et autres dans "Angewandte Chemie", vol 27, n°1, Janvier 1988, pages 129 et 137.

Comme expliqué ci-dessus, on associe le plus souvent au(x) lipide(s) amphiphile(s) au moins un additif stabilisant ayant pour fonction de diminuer la perméabilité de la membrane des vésicules et/ou d'améliorer la stabilité des vésicules.

Parmi ces additifs on peut citer :

- les stérols et leurs dérivés, par exemple oxyéthylénés, plus particulièrement le cholestérol, le sulfate de cholestérol acide et ses sels alcalins et le phosphate de cholestérol acide et ses sels alcalins,
- les alcools et diols à longue chaîne,
- les amines à longue chaîne et leurs dérivés ammonium quaternaire,
- les dihydroxyalkylamines, les amines grasses polyoxyéthylénées ; les esters d'aminoalcools à longue chaîne et leurs sels et dérivés ammonium quaternaire,
- les esters phosphoriques d'alcools gras, par exemple le dicétylphosphate et le dimyristylphosphate sous forme d'acides ou de sels alcalins,
- les alkylsulfates, par exemple le cétylsulfate de sodium,
- certains polymères, tels que les polypeptides et les protéines.

De façon connue, on peut introduire dans la phase lipidique constituant la membrane des vésicules au moins un composé liposoluble cosmétiquement actif. Selon l'invention, on peut utiliser tout actif liposobluble dans la mesure où il est utile dans les compositions pour le bain et où il ne détruit pas la stabilité des vésicules. Parmi les actifs liposolubles utilisables selon l'invention, on peut citer les lipoprotides et, notamment, les lipoprotides exempts de fonction sulfhydryle choisis parmi les dérivés mono- ou polyacylés d'amino-acides ou de polypeptides, dans lesquels le reste acyle $R_{14}$-CO comporte une chaîne hydrocarbonée $R_{14}$ en $C_{13}$-$C_{19}$, au moins une des fonctions qui relie la chaîne polypeptidique ou le reste d'amino-acide à la chaîne lipophile étant une fonction amide, les fonctions carboxyliques de la chaîne polypeptidique ou du reste d'amino-acide pouvant être partiellement ou totalement neutralisées par un ou plusieurs cations alcalins, un ion ammonium ou un ammonium substitué dérivé d'une amine.

La phase lipidique constituant la membrane des vésicules contient généralement de 1 à 80 % et, de préférence, de 10 à 40 % en poids de lipide(s) amphiphile(s) ionique(s) et/ou non-ionique(s).

Dans le cas où la phase encapsulée dans les vésicules est une phase aqueuse, on peut introduire, de façon connue, dans la phase encapsulée au moins un composé hydrosoluble cosmétiquement actif. Selon la présente invention, on

choisira des composés hydrosolubles utiles dans les compositions pour le bain, tels que la glycérine, les complexes de vitamines, les extraits d'algue et le menthol.

Des actifs amphiphiles peuvent se répartir entre la phase lipidique et la phase aqueuse encapsulée ou entre la phase lipidique et la phase aqueuse de dispersion.

La phase aqueuse de dispersion et la phase aqueuse encapsulée sont, de préférence, constituées par de l'eau.

La phase aqueuse de dispersion peut contenir, de façon connue, comme la phase aqueuse encapsulée, au moins un composé hydrosoluble cosmétiquement actif tel que les humectants et les polymères. Elle peut également, de façon connue, contenir une dispersion de gouttelettes, d'au moins un composé non miscible à l'eau, stabilisées par les vésicules de lipide(s) amphiphile(s) ionique(s) et/ou non-ionique(s). Ces composés non miscibles à l'eau peuvent être des huiles minérales ou végétales, éventuellement oxyéthylénées, des huiles essentielles naturelles ou synthétiques, des esters d'acides gras, des mono-, di-ou triglycérides d'acides gras à longue chaîne ou des huiles de silicone.

La phase lipidique constituant la membrane des vésicules représente avantageusement de 1 à 16 %, de préférence de 2 à 12 % en poids par rapport au poids total de la composition. La composition contient avantageusement de 0,5 à 15 %, de préférence de 1 à 11 %, en poids de lipide(s) amphiphile(s) ionique(s) et/ou non-ionique(s) par rapport au poids total de la composition.

L'additif stabilisant de la phase lipidique représente avantageusement moins de 8 %, de préférence de 0,1 à 6 % en poids par rapport au poids total de la composition.

Les vésicules dispersées ont, avantageusement, un diamètre moyen compris entre 10 et 1 000 nm.

La viscosité de la composition est avantageusement comprise entre 20 et 100 Pa.s.

La dispersion de vésicules de lipide(s) amphiphile(s) ionique(s) et/ou non-ionique(s) peut se faire par tout procédé connu. Le composé de formule (I) est ajouté dans la phase aqueuse de dispersion

La dispersion de vésicules de lipide(s) amphiphile(s) ionique(s) et/ou non-ionique(s) peut se faire par tout procédé connu. Le composé de formule (I) est ajouté dans la phase aqueuse de dispersion après préparation des vésicules si on désire que la phase aqueuse de dispersion seule contienne le(s) composé(s) de formule (I). Le(s) composé(s) de formule (I) est (sont) introduit(s) dans la phase aqueuse mise en contact avec la phase lipidique si on désire que ladite phase aqueuse encapsulée contienne un (des) composé(s) de formule (I).

On peut également introduire une partie du (des) composé(s) de formule (I) dans la phase aqueuse mise en contact avec la phase lipidique et ajouter une quantité supplémentaire dans la phase aqueuse de dispersion après réalisation de la dispersion vésiculaire.

On peut également introduire dans la composition des composés n'ayant aucune action cosmétique propre mais qui sont nécessaires pour la formulation, tels que des conservateurs, des parfums, des agents acidifiants ou alcalinisants, des agents stabilisants ou des colorants.

La composition peut être introduite dans l'eau du bain en quantités variables. Des quantités voisines de 1 g par litre d'eau du bain donnent des résultats satisfaisants.

Pour mieux faire comprendre l'objet de l'invention, on va en décrire ci-après, à titre d'exemples purement illustratifs, plusieurs modes de mise en oeuvre.

<u>EXEMPLE 1</u> :

Dans une première étape, on fond, en agitant doucement à une température de 90-95° C, un mélange de 3,8 g de lipide non-ionique de formule

$$C_{16}H_{33}O\left[C_3H_5(OH)O\right]_{\bar{n}}H$$

formule où $\bar{n}$ est une valeur statistique moyenne égale à 3, et où $-C_3H_5(OH)O-$ est représenté par les structures suivantes prises en mélange ou séparément :

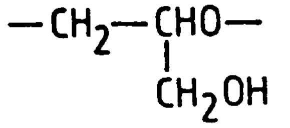

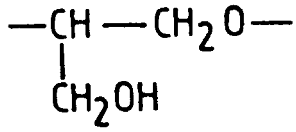

avec 3,8 g de cholestérol et 0,4 g du sel monosodique du glutamate de formule :

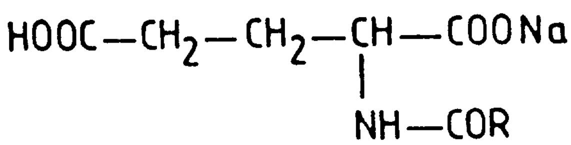

dans laquelle R est un mélange de radicaux alkényle et/ou alkyle hydrogénés en C14-C22 dérivé des acides gras du suif, vendu sous la dénomination commerciale "ACYLGLUTAMATE HS 11"® par la société AJINOMOTO.

On introduit dans le mélange fondu 16 g d'eau portée à 90° C contenant un conservateur et l'on mélange pendant environ 6 à 8 minutes au moyen d'un agitateur "ULTRA TURRAX"®. A la phase ainsi obtenue, on ajoute 24 g d'eau à température ambiante, puis on homogénéise le mélange à l' "ULTRA TURRAX"® pendant encore environ 6 à 8 mn avant de laisser revenir le mélange à la température ambiante.

Dans une seconde étape, on additionne 16 g de propylèneglycol en agitant à l' "ULTRA TURRAX"® pendant 5 mn. Puis on ajoute (sous agitation magnétique) une solution aqueuse renfermant des conservateurs et un colorant pour compléter à 100 g.

Dans la composition, le rapport en poids phase lipidique/composé de formule (I) est de 0,5.

Cette composition présente une bonne stabilité de conservation. Lorsqu'on l'ajoute dans l'eau d'un bain à raison d'environ 1 g par litre d'eau, on constate une parfaite autodispersibilité. L'utilisation régulière de cette composition comme additif d'un bain confère une grande douceur à la peau de l'utilisateur.

EXEMPLE 2 :

On prépare une composition comme dans l'exemple 1 en remplaçant 16 g de propylèneglycol par 8 g de propylèneglycol. Dans la composition, le rapport en poids phase lipidique/composé de formule (I) est de 1. La composition obtenue présente les mêmes avantages que celle de l'exemple 1.

EXEMPLE 3 :

On prépare une composition comme dans l'exemple 1 en remplaçant 16 g de propylèneglycol par 4 g de propylèneglycol. Dans la composition, le rapport en poids phase lipidique/composé de formule (I) est de 2. La composition obtenue présente les mêmes avantages que celle de l'exemple 1.

EXEMPLE 4 :

On prépare une composition comme dans l'exemple 1 en remplaçant le lipide non-ionique de l'exemple 1 par un lipide non-ionique de formule

$$C_{15}H_{31}-CO-\left[OCH_2-CHOH-CH_2\right]_2-OH$$

et en remplaçant 16 g de propylèneglycol par 5,35 g de monoéthyléther de diéthylèneglycol. Dans la composition, le rapport en poids phase lipidique/composé de formule (I) est égal à 1,5. La composition obtenue présente les mêmes avantages que celle de l'exemple 1.

EXEMPLE 5 :

On prépare une composition comme dans l'exemple 1, les huiles essentielles étant ajoutées en même temps que le dipropylèneglycol.

| | | | |
|---|---|---|---|
| Lipide non-ionique de l'exemple 1 | | 3,8 g | } |
| Cholestérol | | 3,8 g | } 8 g |
| "ACYLGLUTAMATE HS 11"® défini dans l'exemple 1 | | 0,4 g | } |
| Dipropylèneglycol | | 8 g | |
| Huiles essentielles | | 1,3 g | |
| Parfum, conservateurs, colorant | qs | | |
| Eau | qsp | 100 g | |

Dans cette composition, le rapport en poids phase lipidique/composé de formule (I) est égal à 1. Cette composition présente les mêmes avantages que celle de l'exemple 1.

EXEMPLE 6 :

Dans une première étape, on fond, en agitant doucement à une température de 80-90°C, 8 g de phase lipidique constituée par un mélange de 3,8 g de lipides non-ioniques de formule

$$C_{12}H_{25}-\left[OC_2H_3(R)\right]-O-\left[C_3H_5-(OH)-O\right]_{\bar{n}}-H$$

dans laquelle :

- $-C_3H_5(OH)O-$
  est constitué par un mélange de radicaux :

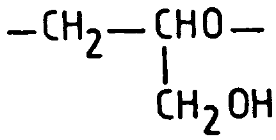 et 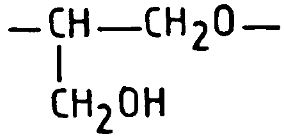

- $-O-C_2H_3(R)-$
  est constitué par un mélange de radicaux :

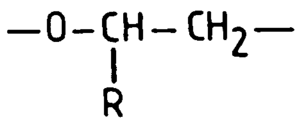 et 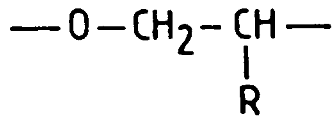

- $\bar{n} = 6$
- R est un mélange de radicaux $C_{14}H_{29}$ et $C_{16}H_{33}$ avec 0,2 g de dimyristylphosphate.

On introduit dans le mélange fondu 8 g d'eau portée à 90° C contenant un conservateur et l'on mélange pendant environ 6 à 8 minutes au moyen d'un agitateur "ULTRA TURRAX"®. A la phase ainsi obtenue, on ajoute 12 g d'eau à température ambiante, puis on homogénéise le mélange à l' "ULTRA TURRAX"® pendant encore environ 6 à 8 minutes avant de laisser revenir le mélange à la température ambiante.

Dans une seconde étape, on additionne 16 g de dipropylèneglycol en agitant à l' "ULTRA TURRAX"® pendant 5 minutes. Puis on ajoute, sous agitation magnétique, une solution aqueuse renfermant des conservateurs et un colorant pour compléter à 100 g.

Dans cette composition, le rapport en poids phase lipidique/composé de formule (I) est égal à 0,5. Cette composition présente les mêmes avantages que celle de l'exemple 1.

<u>EXEMPLE 7</u> :

On prépare une composition comme dans l'exemple 1, les huiles étant ajoutées en même temps que le dipropylèneglycol.

| | | | |
|---|---|---|---|
| Lipide non-ionique de l'exemple 1 | | 3,8 g | } |
| Cholestérol | | 3,8 g | } 8 g |
| "ACYLGLUTAMATE HS 11"® défini dans l'exemple 1 | | 0,4 g | } |
| Dipropylèneglycol | | 7 g | |
| Huiles essentielles | | 0,5 g | |
| Huile de pépins de raisin | | 0,5 g | |
| Huile de graines de tomate | | 0,5 g | |
| Parfum, conservateurs, colorant | qs | | |
| Eau | qsp | 100 g | |

Dans cette composition, le rapport en poids phase lipidique/composé de formule (I) est égal à 1,14. Cette composition présente les mêmes avantages que celle de l'exemple 1.

EXEMPLE 8 :

On prépare une composition comme dans l'exemple 1, les huiles étant ajoutées en même temps que le dipropylèneglycol.

| | | | |
|---|---|---|---|
| Lipide non-ionique de l'exemple 1 | | 3,8 g | } |
| Cholestérol | | 3,8 g | } 8 g |
| Dihexadécylphosphate | | 0,4 g | } |
| Dipropylèneglycol | | 8 g | |
| Huiles essentielles | | 0,14 g | |
| Parfum, conservateurs, colorant | qs | | |
| Eau | qsp | 100 g | |

Dans cette composition, le rapport en poids phase lipidique/composé de formule (I) est égal à 1. Cette composition présente les mêmes avantages que celle de l'exemple 1.

EXEMPLE 9 :

Dans une première étape, on fond, en agitant doucement à une température de 85° C, 1,6 g de phytostérol polyoxyéthyléné à 5 moles d'oxyde d'éthylène vendu par la société NIKKO sous la dénomination "GENEROL 122 E 5"®. Puis au mélange fondu, on additionne 2,4 g de lécithine hydrogénée à 30-35 % en poids de phosphatidylcholine hydrogénée, vendue par la société NIKKO sous la dénomination "LECINOL S 10"®, et ceci jusqu'à homogénéisation parfaite (5 mn).

On introduit dans le mélange fondu 8 g d'eau portée à 80° C contenant un conservateur et l'on mélange pendant environ 5 mn, puis on laisse gonfler le mélange pendant 1 heure. A la phase ainsi obtenue, on ajoute 12 g d'eau à 20° C ; on agite le mélange pendant quelques minutes ; on affine le mélange par un passage dans un homogénéiseur haute pression "GAULIN"® à 500 bars avant de laisser revenir le mélange à température ambiante.

Dans une seconde étape, on additionne 16 g de monométhyléther de propylèneglycol en agitant à l' "ULTRA TURRAX"®. Puis on complète à 100 g par de l'eau contenant un conservateur, sous agitation magnétique.

Dans cette composition, le rapport en poids phase lipidique/composé de formule (I) est égal à 0,25. Cette composition présente les mêmes avantages que celle de l'exemple 1.

**Revendications**

1. Composition cosmétique destinée à être additionnée à l'eau d'un bain pour le soin de la peau, comprenant, dans une phase aqueuse de dispersion, une dispersion de vésicules, dont la membrane est constituée par une phase lipidique contenant au moins un lipide amphiphile ionique et/ou au moins un lipide amphiphile non-ionique, caractérisée par le fait que la phase aqueuse de dispersion contient au moins un composé de formule

$$R'\text{-}O\text{-}[RO]_n\text{-}R'' \qquad (I)$$

formule dans laquelle R est un radical alkylène, linéaire ou ramifié, en $C_2$-$C_3$, R' et R", identiques ou différents, sont H ou un radical alkyle en $C_1$-$C_4$ et n est un nombre entier compris entre 1 et 3 inclus, le rapport pondéral entre la phase lipidique des vésicules et l'ensemble du (ou des) composé(s) de formule (I) contenus dans la phase de dispersion étant compris entre 0,25 et 2,5.

2. Composition selon la revendication 1, caractérisée par le fait que la phase lipidique constituant la membrane des vésicules représente de 1 à 16 % en poids par rapport au poids total de la composition.

3. Composition selon l'une des revendications 1 ou 2, caractérisée par le fait que la membrane lipidique des vésicules encapsule une phase aqueuse, qui contient au moins un composé de formule (I).

**4.** Composition selon l'une des revendications 1 à 3, caractérisé par le fait que le rapport pondéral entre la phase lipidique et le(s) composé(s) de formule (I) est compris entre 0,5 et 1,5.

**5.** Composition selon l'une des revendications 1 à 4, caractérisée par le fait qu'elle contient de 0,5 à 20 % en poids de composé(s) de formule (I) par rapport au poids total de la composition.

**6.** Composition selon l'une des revendications 1 à 5, caractérisée par le fait que les composés de formule (I) sont choisis dans le groupe formé par l'éthylèneglycol, le diéthylèneglycol, le propylèneglycol, le dipropylèneglycol, les éthers monométhylique, monoéthylique et monobutylique de l'éthylèneglycol et du diéthylèneglycol, et les monométhyléthers du propylèneglycol et du dipropylèneglycol.

**7.** Composition selon l'une des revendications 1 à 6 caractérisée par le fait que le(s) lipide(s) non-ionique(s) est (sont) choisi(s) dans le groupe formé par :

(1) les dérivés de polyglycérol, linéaires ou ramifiés, de formule

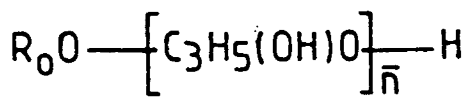 (II)

formule (II) dans laquelle :

- $-C_3H_5(OH)O-$ est représenté par les structures suivantes prises en mélange ou séparément : $-CH_2CHOHCH_2O-$ ,

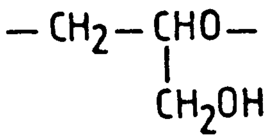 , 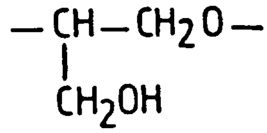

- $\bar{n}$ est une valeur statistique moyenne comprise entre 1 et 6 ;
- $R^0$ représente :

  (a) une chaîne aliphatique, linéaire ou ramifiée saturée ou insaturée, contenant de 12 à 30 atomes de carbone ; ou des radicaux hydrocarbonés des alcools de lanoline ; ou les restes d'alpha-diols à longue chaîne ;
  (b) un reste $R^1CO$, où $R^1$ est un radical aliphatique, linéaire ou ramifié, en $C_{11}$-$C_{29}$ ;
  (c) un reste
  $R_2[OC_2H_3(R_3)]$
  où :

  - $R^2$ peut prendre la signification (a) ou (b) donnée pour $R^0$ ;
  - $-OC_2H_3(R^3)-$
    est représenté par les structures suivantes, prises en mélange ou séparément :

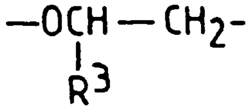 et 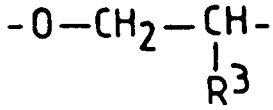

où $R^3$ prend la signification (a) donnée pour $R^o$ ;

(2) les éthers de polyglycérol, linéaires ou ramifiés, comportant deux chaînes grasses ;

(3) les alcools gras polyoxyéthylénés et les stérols et phytostérols polyoxyéthylénés ;
(4) les éthers de polyols ;
(5) les esters de polyols, oxyéthylénés ou non ;
(6) les glycolipides d'origine naturelle ou synthétique et notamment les éthers et les esters de glucose ;
(7) les hydroxyamides représentés par la formule :

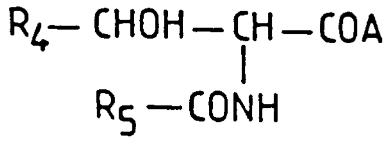

(III)

formule (III) dans laquelle :

- $R^4$ désigne un radical alkyle ou alcényle en $C_7$-$C_{21}$ ;
- $R^5$ désigne un radical hydrocarboné, saturé ou insaturé, en $C_7$-$C_{31}$ ;
- COA désigne un groupement choisi parmi les deux groupements suivants :
  - un reste

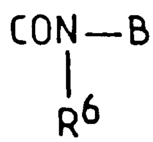

où :

. B est un radical alcoyle dérivé d'amines primaires ou secondaires, mono- ou polyhydroxylées ; et
. $R^6$ désigne un atome d'hydrogène ou un radical méthyle, éthyle ou hydroxyéthyle ; et

  - un reste -COOZ, où Z représente le reste d'un polyol en $C_3$-$C_7$.

(8) les dérivés du glycérol répondant à la formule

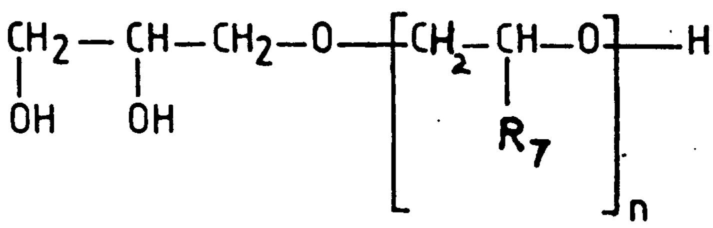

(IV)

formule (IV) dans laquelle $R_7$ représente un radical alkyle linéaire en $C_{14}$-$C_{18}$ ou un groupement $-CH_2A$ dans lequel A est $-OR_{14}$, $R_{14}$ représentant un radical alkyle linéaire en $C_{10}$ à $C_{18}$ et n représente ou bien une valeur statistique moyenne supérieure à 1 et au plus égale à 3, ou bien, lorsque $R_7$ représente $-CH_2A$, une valeur égale à 2.

**8.** Composition selon l'une des revendications 1 à 7, caractérisée par le fait que le(s) lipide(s) amphiphile(s) ionique(s) est (sont) choisi(s) dans le groupe formé par :

- les phospholipides naturels, les phospholipides modifiés par voie chimique ou enzymatique et les phospholipides synthétiques,

- les composés anioniques de formule

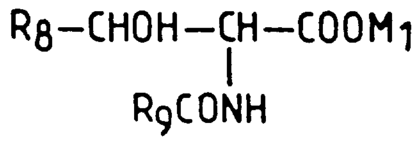 (V)

formule (V) dans laquelle :

- $R_8$ représente un radical alkyle ou alcényle en $C_7$-$C_{21}$
- $R_9$ représente un radical hydrocarboné, saturé ou insaturé en $C_7$-$C_{31}$, et
- M représente H, Na, K, $NH_4$ ou un ion ammonium substitué dérivé d'une amine,

- les gangliosides,
- les composés cationiques ayant la formule :

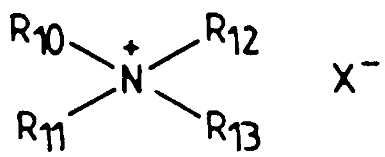 (VI)

avec $R_{10}$ et $R_{11}$, identiques ou différents, représentant des radicaux alkyle en $C_{12}$ - $C_{20}$ et $R_{12}$ et $R_{13}$, identiques ou différents, des radicaux alkyle en $C_1$ - $C_4$ ; et
- les lipides polymérisables.

**9.** Composition selon l'une des revendications 1 à 8, caractérisée par le fait que la phase lipidique constituant la membrane des vésicules contient au moins un additif stabilisant ayant pour fonction de diminuer la perméabilité de la membrane des vésicules et/ou d'améliorer la stabilité des vésicules.

**10.** Composition selon la revendication 9, caractérisée par le fait que l'additif stabilisant est choisi dans le groupe formé par :

- les stérols et leurs dérivés,
- les alcools et diols à longue chaîne,
- les amines à longue chaîne et leurs dérivés ammonium quaternaire,
- les dihydroxyalkylamines, les amines grasses polyoxyéthylénées ; les esters d'aminoalcools à longue chaîne et leurs sels et dérivés ammonium quaternaire,
- les esters phosphoriques d'alcools gras,
- les alkylsulfates,
- des polypeptides et des protéines.

**11.** Composition selon l'une des revendications 1 à 10, caractérisée par le fait que la phase lipidique constituant la membrane des vésicules contient de 1 à 80 % en poids de lipide(s) amphiphile(s).

**12.** Composition selon l'une des revendications 1 à 11, caractérisée par le fait que la phase aqueuse de dispersion contient une dispersion de gouttelettes d'au moins un composé non-miscible à l'eau stabilisées par les vésicules lipidiques.

**13.** Composition selon l'une des revendications 1 à 12, caractérisée par le fait qu'elle contient au moins un additif cosmétiquement acceptable choisi dans le groupe formé par les huiles de silicone, les huiles minérales et végétales oxyéthylénées et non oxyéthylénées, les huiles essentielles naturelles et synthétiques, les esters d'acides gras, les mono-, di- et tri-glycérides d'acide gras à longue chaîne, les polyols en $C_2$-$C_6$, les humectants, les polymères, les conservateurs, les parfums, les agents acidifiants et alcalinisants, les agents stabilisants et les colorants.

**14.** Composition selon l'une des revendications 1 à 13, caractérisée par le fait qu'elle contient de 0,5 à 15 % en poids de lipide(s) amphiphile(s) ionique(s) et/ou non-ionique(s) par rapport au poids total de la composition.

**15.** Composition selon la revendication 9, caractérisée par le fait que l'additif stabilisant de la phase lipidique représente moins de 8 % en poids par rapport au poids total de la composition.

**16.** Composition selon l'une des revendications 1 à 15, caractérisée par le fait que les vésicules ont un diamètre moyen compris entre 10 et 1 000 nm.

**17.** Composition selon l'une des revendications 1 à 16, caractérisée par le fait que sa viscosité est comprise entre 20 et 100 Pa.s.

**18.** Procédé de fabrication d'une composition selon l'une des revendications 1 à 17, caractérisé par le fait que, dans une première étape, on réalise une dispersion de vésicules dans une phase aqueuse de dispersion et que, dans une deuxième étape, on ajoute au moins un composé de formule (I) dans ladite phase aqueuse de dispersion.

**Claims**

**1.** Cosmetic skin care composition for adding to bath water, comprising, in an aqueous dispersion phase, a dispersion of vesicles, the membrane of which consists of a lipid phase containing at least one ionic amphiphilic lipid and/or at least one non-ionic amphiphilic lipid, characterized in that the aqueous dispersion phase contains at least one compound of formula

$$R'-O-(RO)_n-R'' \qquad (I)$$

in which formula R is a linear or branched $C_2$-$C_3$ alkylene radical, R' and R", which may be identical or different, are H or a $C_1$-$C_4$ alkyl radical and n is an integer between 1 and 3 inclusively, the weight ratio between the lipid phase of the vesicles and (all of) the compound(s) of formula (I) contained in the dispersion phase being between 0.25 and 2.5.

**2.** Composition according to Claim 1, characterized in that the lipid phase constituting the vesicle membrane represents from 1 to 16% by weight relative to the total weight of the composition.

**3.** Composition according to either of Claims 1 and 2, characterized in that the lipid membrane of the vesicles encapsulates an aqueous phase which contains at least one compound of formula (I).

**4.** Composition according to one of Claims 1 to 3, characterized in that the weight ratio between the lipid phase and the compound(s) of formula (I) is between 0.5 and 1.5.

**5.** Composition according to one of Claims 1 to 4, characterized in that it contains from 0.5 to 20% by weight of compound(s) of formula (I) relative to the total weight of the composition.

**6.** Composition according to one of Claims 1 to 5, characterized in that the compounds of formula (I) are chosen from the group formed by ethylene glycol, diethylene glycol, propylene glycol, dipropylene glycol, the monomethyl, monoethyl and monobutyl ethers of ethylene glycol and of diethylene glycol, and the monomethyl ethers of propylene glycol and of dipropylene glycol.

**7.** Composition according to one of Claims 1 to 6, characterized in that the non-ionic lipid(s) is (are) chosen from the group formed by:

(1) linear or branched polyglycerol derivatives, of formula

$$R_0O-\left[C_3H_5(OH)O\right]_{\bar{n}}-H \qquad (II)$$

in which formula (II):

- $-C_3H_5(OH)O-$ is represented by the following structures taken as a mixture or separately: $-CH_2CHOHCH_2O-$ ,

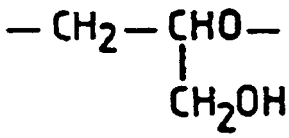 , 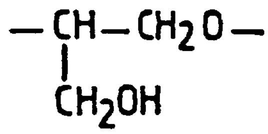

- $\bar{n}$ is an average statistical value between 1 and 6;
- R° represents:

(a) a saturated or unsaturated linear or branched aliphatic chain containing from 12 to 30 carbon atoms; or hydrocarbon radicals of lanolin alcohols; or long-chain alpha-diol residues;
(b) a residue $R^1CO$, where $R^1$ is a linear or branched $C_{11}$-$C_{29}$ aliphatic radical;
(c) a residue
$R_2[OC_2H_3(R_3)]$
where:

- $R^2$ may take the meaning (a) or (b) given for R°;
- $-OC_2H_3(R^3)-$
is represented by the following structures, taken as a mixture or separately:

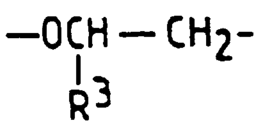 and 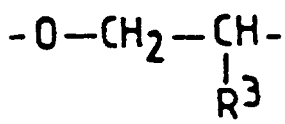

where $R^3$ takes the meaning (a) given for R°;

(2) linear or branched polyglycerol ethers containing two fatty chains;
(3) polyoxyethylenated fatty alcohols and polyoxyethylenated phytosterols and sterols;
(4) polyol ethers;
(5) oxyethylenated or non-oxyethylenated polyol esters;
(6) glycolipids of natural or synthetic origin and in particular glucose ethers and esters;

(7) hydroxy amides represented by the formula:

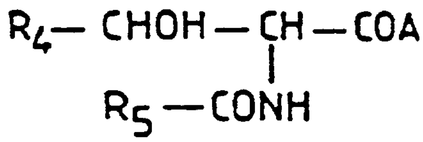

(III)

in which formula (III):

- $R^4$ denotes a $C_7$-$C_{21}$ alkyl or alkenyl radical;
- $R^5$ denotes a $C_7$-$C_{31}$ saturated or unsaturated hydrocarbon radical;
- COA denotes a group chosen from the following two groups:

  - a residue

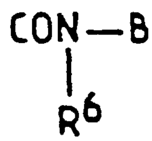

  where:

    - B is an alkyl radical derived from mono- or polyhydroxylated primary or secondary amines; and
    - $R^6$ denotes a hydrogen atom or a methyl, ethyl or hydroxyethyl radical; and

  - a residue -COOZ, where Z represents a $C_3$-$C_7$ polyol residue;

(8) the glycerol derivatives corresponding to the formula:

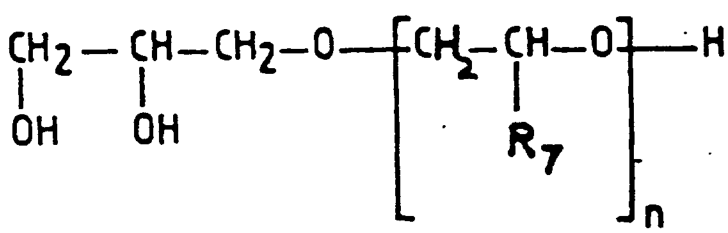

(IV)

in which formula (IV) $R_7$ represents a linear $C_{14}$-$C_{18}$ alkyl radical or a group $-CH_2A$ in which A is $-OR_{14}$, $R_{14}$ representing a linear $C_{10}$ to $C_{18}$ alkyl radical, and n represents either an average statistical value greater than 1 and at least equal to 3, or alternatively, when $R_7$ represents $-CH_2A$, a value equal to 2.

**8.** Composition according to one of Claims 1 to 7, characterized in that the anionic amphiphilic lipid(s) is (are) chosen from the group formed by:

- natural phospholipids, phospholipids modified via a chemical or enzymatic route, and synthetic phospholipids,

- anionic compounds of formula

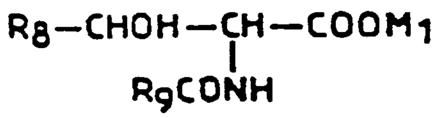

(V)

in which formula (V):

- $R_8$ represents a $C_7$-$C_{21}$ alkyl or alkenyl radical
- $R_9$ represents a saturated or unsaturated $C_7$-$C_{31}$ hydrocarbon radical, and
- M represents H, Na, K, $NH_4$ or a substituted ammonium ion derived from an amine,

- gangliosides,
- cationic compounds having the formula:

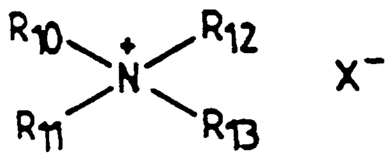

(VI)

with $R_{10}$ and $R_{11}$, which may be identical or different, representing $C_{12}$ - $C_{20}$ alkyl radicals and $R_{12}$ and $R_{13}$, which may be identical or different, representing $C_1$ - $C_4$ alkyl radicals; and
- polymerizable lipids.

**9.** Composition according to one of Claims 1 to 8, characterized in that the lipid phase constituting the vesicle membrane contains at least one stabilizing additive having the function of decreasing the permeability of the vesicle membrane and/or of improving the stability of the vesicles.

**10.** Composition according to Claim 9, characterized in that the stabilizing additive is chosen from the group formed by:

- sterols and their derivatives,
- long-chain alcohols and diols,
- long-chain amines and quaternary ammonium derivatives thereof,
- dihydroxyalkylamines, polyoxyethylenated fatty amines; esters of long-chain amino alcohols and salts and quaternary ammonium derivatives thereof,
- phosphoric esters of fatty alcohols,
- alkyl sulphates,
- polypeptides and proteins.

**11.** Composition according to one of Claims 1 to 10, characterized in that the lipid phase constituting the vesicle membrane contains from 1 to 80% by weight of amphiphilic lipid(s).

**12.** Composition according to one of Claims 1 to 11, characterized in that the aqueous dispersion phase contains a dispersion of droplets of at least one water-immiscible compound which are stabilized by the lipid vesicles.

**13.** Composition according to one of Claims 1 to 12, characterized in that it contains at least one cosmetically acceptable additive chosen from the group formed by silicone oils, oxyethylenated and non-oxyethylenated mineral and vegetable oils, natural and synthetic essential oils, fatty acid esters, mono-, di- and triglycerides of long-chain fatty acids, $C_2$-$C_6$ polyols, moisturizing agents, polymers, preservatives, perfumes, acidifying and basifying agents, stabilizers and colouring agents.

**14.** Composition according to one of Claims 1 to 13, characterized in that it contains from 0.5 to 15% by weight of ionic and/or non-ionic amphiphilic lipid(s) relative to the total weight of the composition.

**15.** Composition according to Claim 9, characterized in that the stabilizing additive of the lipid phase represents less than 8% by weight relative to the total weight of the composition.

**16.** Composition according to one of Claims 1 to 15, characterized in that the vesicles have an average diameter between 10 and 1,000 nm.

**17.** Composition according to one of Claims 1 to 16, characterized in that its viscosity is between 20 and 100 Pa.s.

**18.** Process for the manufacture of a composition according to one of Claims 1 to 17, characterized in that, in a first step, a dispersion of vesicles in an aqueous dispersion phase is prepared and in that, in a second step, at least one compound of formula (I) is added to the said aqueous dispersion phase.

**Patentansprüche**

**1.** Kosmetisches Mittel als Badewasserzusatz zur Pflege der Haut, enthaltend in einer wäßrigen Dispersionsphase eine Dispersion von Vesikeln, deren Membran aus einer Lipidphase gebildet wird, welche wenigstens ein ionisches amphiphiles und/oder wenigstens ein nicht-ionisches amphiphiles Lipid enthält, dadurch gekennzeichnet, daß die wäßrige Dispersionsphase wenigstens eine Verbindung der Formel

$$R'\text{-}O\text{-}[RO]_n\text{-}R'' \qquad (I)$$

enthält,
worin R für einen linearen oder verzweigten $C_2$-$C_3$-Alkylenrest steht, R' und R", die gleich oder verschieden sind, für H oder einen $C_1$-$C_4$-Alkylrest stehen und n für eine ganze Zahl von 1 bis 3 einschließlich steht und wobei das Gewichtsverhältnis zwischen der Vesikellipidphase und der Gesamtheit der in der Dispersionsphase enthaltenen Verbindung(en) der Formel (I) zwischen 0,25 und 2,5 liegt.

**2.** Mittel nach Anspruch 1, dadurch gekennzeichnet, daß die die Vesikelmembran bildende Lipidphase 1 bis 16 Gew.-%, bezogen auf das Gesamtgewicht des Mittels, ausmacht.

**3.** Mittel nach einem der Ansprüche 1 oder 2, dadurch gekennzeichnet, daß die Vesikellipidmembran eine wäßrige Phase einkapselt, welche wenigstens eine Verbindung der Formel (I) enthält.

**4.** Mittel nach einem der Ansprüche 1 bis 3, dadurch gekennzeichnet, daß das Gewichtsverhältnis zwischen der Lipidphase und der (den) Verbindung(en) der Formel (I) zwischen 0,5 und 1,5 liegt.

**5.** Mittel nach einem der Ansprüche 1 bis 4, dadurch gekennzeichnet, daß es 0,5 bis 20 Gew.-% der Verbindung(en) der Formel (I), bezogen auf das Gesamtgewicht des Mittels, enthält.

**6.** Mittel nach einem der Ansprüche 1 bis 5, dadurch gekennzeichnet, daß die Verbindungen der Formel (I) ausgewählt sind unter Ethylenglykol, Diethylenglykol, Propylenglykol, Dipropylenglykol, dem Monomethyl-, Monoethyl- und Monobutylether des Ethylenglykols und des Diethylenglykols, und Monomethylethern des Propylenglykols und des Dipropylenglykols.

**7.** Mittel nach einem der Ansprüche 1 bis 6, dadurch gekennzeichnet, daß das nicht-ionische Lipid (die nicht-ionischen Lipide) ausgewählt ist (sind) unter:

(1) linearen oder verzweigten Polyglycerinderivaten der Formel

$$R_0O-[C_3H_5(OH)O]_{\bar{n}}-H \qquad (II)$$

worin:

- $-C_3H_5(OH)O-$ für die folgenden Strukturen in Kombination oder einzeln steht: $-CH_2CHOHCH_2O-$ ,

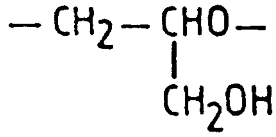 , 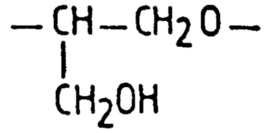

- $\overline{n}$ für einen statistischen Mittelwert zwischen 1 und 6 steht;
- $R^0$ bedeutet:

  (a) eine gesättigte oder ungesättigte, lineare oder verzweigte aliphatische Kette mit 12 bis 30 Kohlenstoffatomen; Kohlenwasserstoffreste von Lanolinalkoholen; oder Reste von langkettigen alpha-Diolen;
  (b) einen Rest $R^1CO$, worin $R^1$ für einen linearen oder verzweigten aliphatischen $C_{11}$-$C_{29}$-Rest steht;
  (c) einen Rest
  $R_2[OC_2H_3(R_3)]$
  worin:

  - $R^2$ die für $R^0$ angegebene Bedeutung (a) oder (b) annehmen kann;
  - $-OC_2H_3(R^3)-$
    für die folgenden Strukturen in Kombination oder einzeln steht:

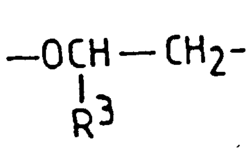 und 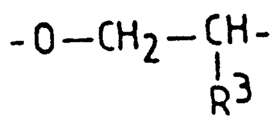

    worin $R^3$ die für $R^0$ angegebene Bedeutung (a) hat;

(2) linearen oder verzweigten Polyglycerinethern mit zwei Fettketten;
(3) polyoxyethylenierten Fettalkoholen und polyoxyethylenierten Sterolen und Phytosterolen;
(4) Polyolethern;
(5) oxyethylenierten oder nicht-oxyethylenierten Polyolestern;
(6) Glykolipiden natürlicher oder synthetischer Herkunft, und insbesondere Glucoseethern und -estern;
(7) Hydroxyamiden der Formel:

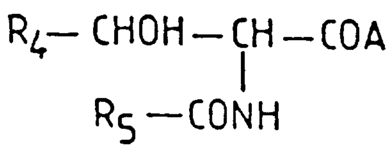 (III)

worin:

- $R^4$ einen $C_7$-$C_{21}$-Alkyl- oder -Alkenylrest bedeutet;
- $R^5$ einen gesättigten oder ungesättigten $C_7$-$C_{31}$-Kohlenwasserstoffrest bedeutet;
- COA für eine Gruppe steht, die ausgewählt ist unter den beiden Gruppen:

- einem Rest

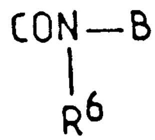

worin:

- B für einen von mono- oder polyhydroxylierten, primären oder sekundären Aminen abgeleiteten Alkylrest steht; und
- $R^6$ ein Wasserstoffatom oder einen Methyl-, Ethyl- oder Hydroxyethylrest bedeutet; und

- einem Rest-COOZ, worin Z für den Rest eines $C_3$-$C_7$-Polyols steht;

(8) Glycerinderivaten der Formel:

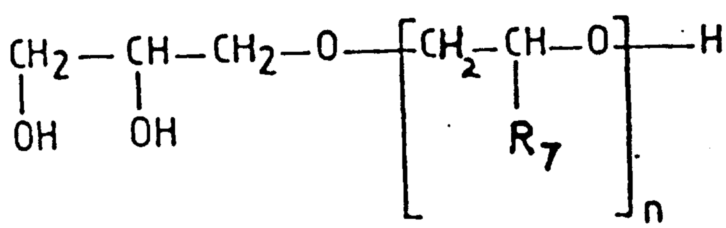

(IV)

worin $R_7$ einen linearen $C_{14}$-$C_{18}$-Alkylrest oder eine Gruppe $-CH_2A$ bedeutet, worin A für $-OR_{14}$ steht, wobei $R_{14}$ einen linearen $C_{10}$-$C_{18}$-Alkylrest bedeutet und n für einen statistischen Mittelwert größer als 1 und höchstens gleich 3 steht oder einen Wert von 2 bedeuten kann, wenn $R_7$ für $-CH_2A$ steht.

**8.** Mittel nach einem der Ansprüche 1 bis 7, dadurch gekennzeichnet, daß das ionische amphiphile Lipid (die ionischen amphiphilen Lipide) ausgewählt ist (sind) unter:

- natürlichen Phospholipiden, auf chemischem oder enzymatischem Weg modifizierten Phospholipiden und synthetischen Phospholipiden;
- anionischen Verbindungen der Formel

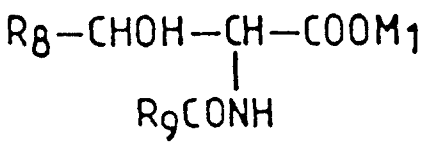

(V)

worin

- $R_8$ für einen $C_7$-$C_{21}$-Alkyl- oder -Alkenylrest steht,
- $R_9$ für einen gesättigten oder ungesättigten $C_7$-$C_{31}$-Kohlenwasserstoffrest steht, und
- M für H, Na, K, $NH_4$ oder ein von einem Amin abgeleitetes substituiertes Ammoniumion steht;

- Gangliosiden;

- kationischen Verbindungen der Formel:

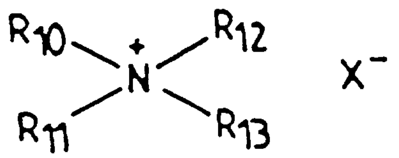

(VI)

wobei $R_{10}$ und $R_{11}$, die gleich oder verschieden sind, für $C_{12}$-$C_{20}$-Alkylreste stehen und $R_{12}$ und $R_{13}$, die gleich oder verschieden sind, für $C_1$-$C_4$-Alkylreste stehen; und

- polymerisierbaren Lipiden.

**9.** Mittel nach einem der Ansprüche 1 bis 8, dadurch gekennzeichnet, daß die die Vesikelmembran bildende Lipidphase wenigstens ein Stabilisierungsadditiv enthält, welches dazu dient, die Permeabilität der Vesikelmembran zu verringern und/oder die Stabilität der Vesikel zu verbessern.

**10.** Mittel nach Anspruch 9, dadurch gekennzeichnet, daß das Stabilisierungsadditiv ausgewählt ist unter:

- Sterolen und ihren Derivaten,
- langkettigen Alkoholen und Diolen,
- langkettigen Aminen und ihren quaternären Ammoniumderivaten,
- Dihydroxyalkylaminen, polyoxyethylenierten Fettaminen; Estern von Aminoalkoholen mit langer Kette und ihren Salzen sowie quaternären Ammoniumderivaten,
- Phosphorsäureestern von Fettalkoholen,
- Alkylsulfaten,
- Polypeptiden und Proteinen.

**11.** Mittel nach einem der Ansprüche 1 bis 10, dadurch gekennzeichnet, daß die die Vesikelmembran bildende Lipidphase 1 bis 80 Gew.-% eines amphiphilen Lipides (amphiphiler Lipide) enthält.

**12.** Mittel nach einem der Ansprüche 1 bis 11, dadurch gekennzeichnet, daß die wäßrige Dispersionsphase eine Dispersion aus Tröpfchen wenigstens einer nicht mit Wasser mischbaren, durch die Lipidvesikel stabilisierten Verbindung enthält.

**13.** Mittel nach einem der Ansprüche 1 bis 12, dadurch gekennzeichnet, daß es wenigstens ein kosmetisch verträgliches Additiv enthält, welches ausgewählt ist unter Silikonölen, oxyethylenierten oder nicht-oxyethylenierten pflanzlichen Ölen und Mineralölen, natürlichen und synthetischen essentiellen Ölen, Estern von Fettsäuren, Mono-, Di- und Triglyceriden von langkettigen Fettsäuren, $C_2$-$C_6$-Polyolen, Befeuchtungsmitteln, Polymeren, Konservierungsmitteln, Parfums, Ansäuerungs- und Alkalinisierungsmitteln, Stabilisierungsmitteln und Färbemitteln.

**14.** Mittel nach einem der Ansprüche 1 bis 13, dadurch gekennzeichnet, daß es 0,5 bis 15 Gew.-% eines ionischen und/oder nicht-ionischen amphiphilen Lipides (ionischer und/oder nicht-ionischer amphiphiler Lipide), bezogen auf das Gesamtgewicht des Mittels, enthält.

**15.** Mittel nach Anspruch 9, dadurch gekennzeichnet, daß das Stabilisierungsadditiv der Lipidphase wenigstens 8 Gew.-%, bezogen auf das Gesamtgewicht des Mittels, ausmacht.

**16.** Mittel nach einem der Ansprüche 1 bis 15, dadurch gekennzeichnet, daß die Vesikel einen mittleren Durchmesser zwischen 10 und 1000 nm besitzen.

**17.** Mittel nach einem der Ansprüche 1 bis 16, dadurch gekennzeichnet, daß seine Viskosität zwischen 20 und 100 Pa.s. liegt.

**18.** Verfahren zur Herstellung eines Mittels nach einem der Ansprüche 1 bis 17, dadurch gekennzeichnet, daß in einem ersten Schritt eine Vesikeldispersion in einer wäßrigen Dispersionsphase hergestellt wird und in einem zweiten Schritt wenigstens eine Verbindung der Formel (I) in die wäßrige Dispersionsphase gegeben wird.